# EUROPÄISCHE PATENTSCHRIFT

(11) **EP 0 569 701 B1**
(45) Veröffentlichungstag und Bekanntmachung des Hinweises auf die Patenterteilung: **27.09.2000**
(21) Anmeldenummer: 93105509.9
(22) Anmeldetag: 02.04.1993
(51) Int. Cl.: C07D 213/73, C07D 213/74

(54) **Verfahren zur Herstellung von 2-Amino-5-methyl-pyridin**
Process for preparation of 2-amino-5-methyl-pyridine
Procédé pour la préparation de amino-2-méthyl-5-pyridine

(30) Priorität: 15.04.1992 DE 4212596; 25.09.1992 DE 4232175
(43) Veröffentlichungstag der Anmeldung: 18.11.1993
(73) Patentinhaber: BAYER AG, 51368 Leverkusen (DE)
(72) Erfinder: Rivadeneira, Eric, W-5090 Leverkusen 3 (DE); Jelich, Klaus, Dr., W-5600 Wuppertal (DE)

(56) Entgegenhaltungen:
- US-A- 4 386 209
- US-A- 4 405 790
- THE JOURNAL OF ORGANIC CHEMISTRY Bd. 39, Nr. 13, 28. Juni 1974, COLUMBUS OHIO Seiten 1795 - 1802 R. A. ABRAMOVITCH ET AL. 'Direct Acylamination of Pyridine 1-Oxides'
- THE JOURNAL OF ORGANIC CHEMISTRY Bd. 39, Nr. 13, 28. Juni 1974, COLUMBUS OHIO Seiten 1802 - 1807 R.A.ABRAMOVITCH ET AL. 'Direct Acylamination of 3-substituted Pyridine 1-Oxides. Directive Effect of the Substituent.'
- JOURNAL OF THE AMERICAN CHEMICAL SOCIETY Bd. 91, Nr. 20, 24. September 1969, COLUMBUS OHIO Seiten 5672 - 5673 R. A. ABRAMOVITCH ET AL. 'A Direct alkyl and aryl amination of Heteroaromatic Nitrogen Compounds'
- CHEM. PHARM. BULL. Bd. 28, Nr. 2, 1980, JAPAN Seiten 465 - 472 KAZUYUKI WACHI ET AL. 'Studies on 1,3-Benzoxazines. I.'
- CHEMICAL ABSTRACTS, Band 53, Nr. 20, 25-10-1959 (Columbus, Ohio, US), Seite , Spalte , Zusammenfassung Nr. 18951d, HANS FüRST ET AL.: "Alkylamino pyridines and alkyl pyridine ethers"

## Beschreibung

Die Erfindung betrifft ein neues Verfahren und neue Zwischenprodukte zur Herstellung von 2-Amino-5-methyl-pyridin.

Es ist bekamt, daß man 2-Amino-5-methyl-pyridin erhält, wenn man 3-Methyl-pyridin mit Natriumamid in einem inerten Verdünnungsmittel bei erhöhter Temperatur und erhöhtem Druck umsetzt (vgl. US-P 4386209).

Weiter ist bekannt, daß man 2-Amino-5-methyl-pyridin erhält, wenn man 3-Methyl-pyridin mit einem Alkylamin-Natriumsalz in einem inerten Verdünnungsmittel umsetzt und das hierbei gebildete 2-Alkylamino-5-methyl-pyridin mit Hydrogenbromid oder Hydrogeniodid entalkyliert (vgl. US-P 4405790).

Bei beiden bekannten Verfahren muß - zumindest in der Anfangsphase der Umsetzungen - wegen der hohen Wasserempfindlichkeit von Natriumamid bzw. Alkylamin-Natriumsalzen eine strikte Trockenheit aller Komponenten gewährleistet sein. Einen weiteren sehr großen Kostenfaktor - insbesondere wegen der zu erfüllenden Sicherheitsauflagen - stellt das jeweils als Ausgangsbasis benötigte metallische Natrium dar.

Die Entstehung von Aminopyridinen durch Umsetzung von Pyridin-N-oxiden mit Imidoylchloriden war bekannt aus J.Org.Chem. Vol. 39 No. 13 S.1795 ff., J.Org.Chem. Vol. 39 No. 13 S.1802ff und J.Am.Chem.Soc. Vol. 91 No. 20 S.5672ff.. Zur Durchführung dieser Reaktion muß jedoch auf nicht ohne weiteres erhältliche Imidoylchloride zurückgegriffen werden.

Es wurde nun ein neues Verfahren zur Herstellung von 2-Amino-5-methyl-pyridin der Formel (I) 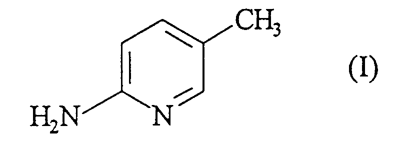 gefunden, welches dadurch gekennzeichnet ist, daß man in einer ersten Stufe 3-Methyl-pyridin-1-oxid der Formel (II) 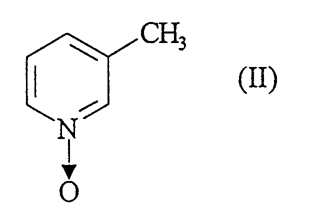 mit einem Trialkylamin der allgemeinen Formel (III)

R₃N (III)

in welcher
- R: für c₁₋₄-Alkyl steht,
und mit Säurechloriden oder Säureanhydriden als elektrophile Verbindung,gegebenenfalls in Gegenwart eines Verdünnungsmittel,
zum Ammoniumsalz der allgemeinen Formel (IV) 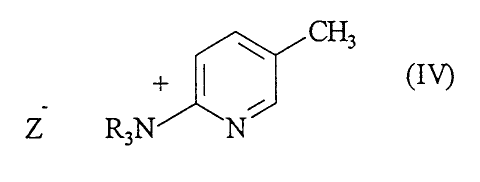 in welcher
- R: die oben angegebene Bedeutung hat und
- Z⁻: für ein aus Säurechlorid oder Säureanhydrid als elektrophile Verbindung gebildetes Anion steht,
umsetzt und die Verbindung (IV)
gegebenenfalls als Rohzwischenprodukt isoliert und gegebenenfalls weiter reinigt
und dann in einer zweiten Stufe
a) entweder das Ammoniumsalz der Formel (IV) mit Hydrogenchlorid oder Hydrogenbromid, gegebenenfalls in Gegenwart eines Verdünnungsmittels, bei Temperaturen zwischen 150°C und 300°C umsetzt,
b) oder alternativ aus der Verbindung (IV) nach üblichen Methoden eine Alkylverbindung RZ abspaltet und das hierbei gebildete 2-Dialkylamino-5-methyl-pyridin der allgemeinen Formel (V) 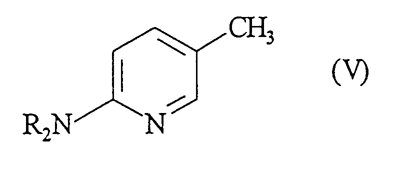 in welcher
   - R: die oben angegebene Bedeutung hat,
   mit Hydrogenchlorid oder Hydrogenbromid, gegebenenfalls in Gegenwart eines Verdünnungsmittels, bei Temperaturen zwischen 150°C und 300°C umsetzt.

Überraschenderweise kann nach dem erfindungsgemäßen Verfahren 2-Amino-5-methyl-pyridin der Formel (I) in hoher Ausbeute und in erheblich verbesserter Isomerenreinheit im Vergleich mit den bekannten Verfahren erhalten werden. Außerdem kann beim erfindungsgemäßen Verfahren mit preiswerten und leicht zugänglichen Ausgangsmaterialien gearbeitet werden.

Die 2-Dialkylamino-5-methyl-pyridine der Formel (V), in welcher R für C₁-C₄-Alkyl steht, sind noch nicht aus der Literatur bekannt und sind als neue chemische Verbindungen Gegenstand der vorliegenden Patentanmeldung. Von den neuen Verbindungen der Formel (V) werden 2-Dimethylamino-5-methyl-pyridin und 2-Diethylamino-5-methyl-pyridin besonders bevorzugt.

In der ersten Stufe des erfindungsgemäßen Verfahrens wird eine elektrophile Verbindung eingesetzt. Bevorzugte elektrophile Verbindungen für das erfindungsgemäße Verfahren sind Säurechloride und Säureanhydride, beispielsweise Acetylchlorid, Propionylchlorid, Acetanhydrid, Propionsäureanhydrid, Benzoylchlorid, Benzotrichlorid, Phosgen, Oxalylchlorid, Benzolsulfochlorid, p-Toluolsulfonsäurechlorid, Phosphor(III)-chlorid, Phosphorylchlorid (Phosphoroxychlorid), Phosphor(V)-chlorid, Thionylchlorid, Sulfurylchlorid, Dichlormethylen-dimethylimmoniumchlorid, Cyanurchlorid und Chlortrimethylsilan.
Phosgen, Thionylchlorid, Sulfurylchlorid, Benzolsulfonsäurechlorid und p-Toluolsulfonsäurechlorid werden als elektrophile Einsatzstoffe für das erfindungsgemäße Verfahren besonders bevorzugt.

Das erfindungsgemäße Verfahren wird in der ersten Stufe vorzugsweise unter Verwendung eines Verdünnungsmittels durchgeführt. Als Verdünnungsmittel kommen praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Die erste Stufe des erfindungsgemäßen Verfahrens wird, falls Trimethylamin als Verbindung (III) eingesetzt wird, im allgemeinen im Temperaturbereich zwischen -30°C und +120°C, vorzugsweise zwischen -15°C und +80°C durchgeführt. Falls andere Trialkylamine (III) als Trimethylamin eingesetzt werden, wird im allgemeinen im Temperaturbereich von -30°C bis +15°C, vorzugsweise zwischen -15°C und +10°C gearbeitet.

Die erste Stufe des erfindungsgemäßen Verfahrens wird im allgemeinen bei Normaldruck durchgeführt. Es ist jedoch auch möglich, unter erhöhtem oder vermindertem Druck, im allgemeinen zwischen 0,1 und 100 bar, zu arbeiten.

Zur Durchführung der ersten Stufe des erfindungsgemäßen Verfahrens setzt man auf 1 Mol 3-Methyl-pyridin-1-oxid der Formel (II) im allgemeinen zwischen 1 und 10 Mol, vorzugsweise zwischen 2 und 5 Mol, Trialkylamin der Formel (III) und zwischen 1 und 10 Mol, vorzugsweise zwischen 2 und 5 Mol, elektrophile Verbindung ein.

In einer bevorzugten Ausführungsform der ersten Stufe des erfindungsgemäßen Verfahrens wird das 3-Methyl-pyridin-1-oxid der Formel (II) in einem Verdünnungsmittel vorgelegt und nach Abkühlen werden nacheinander zuerst das Trialkylamin und dann die elektrophile Verbindung langsam unter Rühren eindosiert.

Nach dem Ende der Umsetzung werden gegebenenfalls die leichter flüchtigen Komponenten unter vermindertem Druck abdestilliert. Das Roh-Zwischenprodukt, welches im wesentlichen das Ammoniumsalz der Formel (IV) enthält, kann auf übliche Weise, z.B. durch Säulenchromatographie gereinigt oder auch roh in die zweite Stufe eingesetzt werden.

Vorzugsweise wird das Roh-Zwischenprodukt als solches in die zweite Stufe eingesetzt.

Die zweite Stufe des erfindungsgemäßen Verfahrens wird gegebenenfalls unter Verwendung eines Verdünnungsmittels durchgeführt. Als Verdünnungsmittel kommen neben Wasser auch organische Lösungsmittel, insbesondere Alkohole wie Methanol, Ethanol oder Propanol, oder Carbonsäuren wie Essigsäure oder Propionsäure, in Betracht.

Die zweite Stufe des erfindungsgemäßen Verfahrens wird im allgemeinen im Temperaturbereich zwischen 150°C und 300°C, vorzugsweise zwischen 180°C und 250°C, insbesondere zwischen 190°C und 220°C, durchgeführt.

Die zweite Stufe des erfindungsgemäßen Verfahrens wird im allgemeinen unter Normaldruck durchgeführt. Es ist jedoch auch möglich, unter erhöhtem oder vermindertem Druck, im allgemeinen zwischen 0,01 und 200 bar, zu arbeiten.

Zur Durchführung der zweiten Stufe des erfindungsgemäßen Verfahrens werden vorzugsweise das Zwischenprodukt der Formel (IV) und eine Lösung von Hydrogenchlorid oder Hydrogenbromid bei Raumtemperatur (ca. 20°C) oder unter Eiskühlung vermischt. Dann wird gegebenenfalls das Lösungsmittel abdestilliert und das Reaktionsgemisch wird auf eine für die Umsetzung geeignete Temperatur aufgeheizt, wobei gegebenenfalls weiter Hydrogenchlorid oder Hydrogenbromid(lösung) eindosiert und gegebenenfalls Lösungsmittel abdestilliert wird. Vorteilhafterweise wird das Erhitzen der Mischung einige Male unterbrochen und der "Ansatz" entspannt, d.h. von Überdruck auf Normaldruck gebracht, um das als Koppelprodukt gebildete Alkylhalogenid zu entfernen.

Nach dem durch Dünnschichtchromatographie ermittelbaren Ende der Umsetzung wird das Gemisch abgekühlt und nach üblichen Methoden aufgearbeitet.

Beispielsweise wird mit verdünnter wäßriger Natronlauge und einem mit Wasser praktisch nicht mischbarem organischen Lösungsmittel, wie z.B. Essigsäureethylester durchgeschüttelt, die organische Phase abgetrennt, getrocknet und filtriert. Vom Filtrat wird das Lösungsmittel unter vermindertem Druck sorgfältig abdestilliert. Es verbleibt ein Rückstand, welcher im wesentlichen das Produkt der Formel (I) enthält.

Alternativ kann in der zweiten Verfahrensstufe zunächst aus den Ammoniumsalzen der Formel (IV) nach üblichen Methoden eine Alkylverbindung RZ unter Bildung von 2-Dialkylamino-5-methyl-pyridinen der Formel (V) abgespaltet werden. Als übliche Methoden hierfür seien beispielsweise genannt:
(a) Erwärmen mit einer Säure, beispielsweise mit einer Hydrogenchlorid-oder Hydrogenbromidlösung, auf mäßig erhöhte Temperatur, vorzugsweise zwischen 50°C und 100°C;
(b) erwärmen mit einer Base, beispielsweise mit wäßriger Natronlauge, auf mäßig erhöhte Temperaturen, vorzugsweise zwischen 50°C und 150°C, gegebenenfalls bei erhöhtem Druck bis zu 10 bar;
(c) erwärmen mit einem Lösungsmittel, beispielsweise mit Dimethylsulfoxid auf mäßig erhöhte Temperatur, vorzugsweise zwischen 80°C und 150°C.

Die Aufarbeitung kann jeweils wie oben beschrieben durchgeführt werden.

Die Entalkylierung der Zwischenprodukte der Formel (V) kann dann analog zur Entalkylierung der Ammoniumsalze der Formel (IV) mit Hydrogenbromid oder Hydrogenchlorid wie oben beschrieben zur Herstellung der Verbindung der Formel (I) durchgeführt werden (vgl. die Herstellungsbeispiele).

Das nach dem erfindungsgemäßen Verfahren herstellbare 2-Amino-5-methyl-pyridin kann als Zwischenprodukt zur Herstellung von Agrochemikalien, beispielsweise von Herbiziden (vgl. EP-A 432600) verwendet werden.

### Herstellungsbeispiele:

### Beispiel 1

### (1. Stufe)

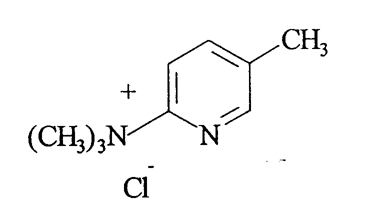

90 g (0,825 Mol) 3-Methyl-pyridin-1-oxid werden in 540 ml Methylenchlorid vorgelegt und das Gemisch wird auf 0°C abgekühlt. Bei dieser Temperatur werden 195 g (3,3 Mol) Trimethylamin einkondensiert. Dann werden 245 g (2,63 Mol) Phosgen eingeleitet, wobei die Temperatur durch intensives Kühlen bei 0°C gehalten wird. Nach Beendigung der Umsetzung wird überschüssiges Phosgen bei 20°C im Wasserstrahlvakuum entfernt. Der verbleibende Rückstand wird durch Säulenchromatographie (Kieselgel; Eluens: Methylenchlorid/Methanol, Vol. 10:1,5) gereinigt.

Man erhält 188 g eines Produktgemisches, welches gemäß ¹H-NMR-Spektrum 102 g (66% der Theorie) Trimethyl-(5-methyl-pyridin-2-yl)-ammoniumchlorid enthält.
- NMR-Daten:: ¹H-NMR (300 MHz, D₆-DMSO) δ/ppm = 2,41 (3H, Singulett); 3,64 (9H, Singulett); 8,06 (2H, AB-System, J_{AB} = 8,4 Hz); 8,5 (1H, Singulett)

### Beispiel 2

### (1. Stufe)

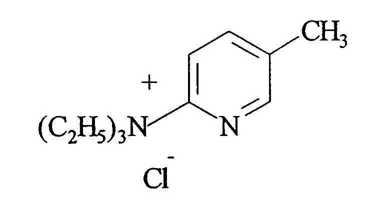

90 g (0,825 Mol) 3-Methyl-pyridin-1-oxid werden in 540 ml Methylenchlorid vorgelegt und das Gemisch wird auf 0°C abgekühlt. Bei dieser Temperatur werden 334 g (3,3 Mol) Triethylamin zugegeben. Dann werden 245 g (2,63 Mol) Phosgen eingeleitet, wobei die Temperatur durch intensives Kühlen bei 0°C gehalten wird. Nach Beendigung der Umsetzung wird überschüssiges Phosgen bei 20°C im Wasserstrahlvakuum entfernt. Der verbleibende Rückstand wird durch Säulenchromatographie (Kieselgel, Eluens: Methylenchlorid/Methanol, Vol. 10: 1,5) gereinigt.

Man erhält 173 g eines Produktgemisches, welches gemäß ¹H-NMR-Spektrum 104 g (55% der Theorie) Triethyl-(5-methyl-pyridin-2-yl)-ammoniumchlorid enthält.
¹H-NMR (300 MHz, CDCl₃)
δ/ppm = 1,20 (9H, Triplett, J = 7,2 Hz), 2,44 (3H, Sin-gulett), 4,14 (6H, Quartett, J = 7,2 Hz), 7,98 (1H, Multiplett, J_{AB} = 8,4 Hz), 8,34 (1H, Quartett, J = 0,9 Hz), 8,63 (1H, Dublett, J_{AB} = 8,7 Hz)

### Beispiel 3

### (1. Stufe)

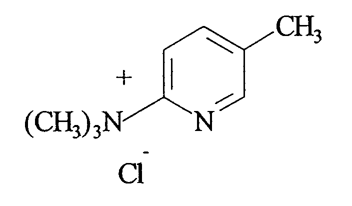

19,5 g (0,331 Mol) Trimethylamin werden bei -10°C kondensiert und bei -5°C zu einer Lösung von 8,8 g (0,0807 Mol) 3-Methyl-pyridin-1-oxid in 100 ml Methylenchlorid gegeben. Dann werden bei dieser Temperatur 6,9 ml (0,097 Mol) Thionylchlorid in 10 ml Methylenchlorid innerhalb 30 Minuten eingetropft. Die Temperatur wird während dieses Vorgangs unter 0°C gehalten. Die resultierende gelbe Lösung läßt man auflauen und über Nacht bei Raumtemperatur nachrühren.

Man erhält eine goldgelbe Lösung, die bei 20-25°C am Rotationsverdampfer eingeengt wird. Es fallen 24,5 g eines schmierigen Feststoffes an, der laut ¹H-NMR ca. 14,5 g (96% der Theorie) Trimethyl-(5-methyl-pyridin-2-yl)-ammoniumchlorid enthält.
¹H-NMR: (300 MHz, D₆-DMSO)
δ/ppm = 2,41 (3H, Singulett); 3,64 (9H, Singulett), 8,06 (2H, AB-System, J_{AB} = 8,4 Hz), 8,5 (1H, Singulett)

### Beispiel 4

### (1. Stufe)

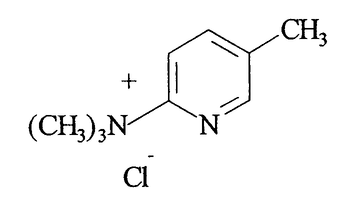

20,5 g (0,348 Mol) Trimethylamin werden bei -10°C kondensiert und anschließend bei -5°C zu einer Lösung von 8,8 g (0,0807 Mol) 3-Methyl-pyridin-1-oxid in 110 ml Methylenchlorid gegeben. Dann wird bei dieser Temperatur eine Lösung von 21,3 ml (0,2421 Mol) Sulfurylchlorid in 20 ml Methylenchlorid eingetropft. Es bildet sich ein weißer, feiner Niederschlag. Man läßt innerhalb einer Stunde auf Raumtemperatur auftauen und anschließend noch 2 Stunden bei dieser Temperatur nachrühren. Der Niederschlag löst sich in dieser Zeit vollständig auf Die resultierende goldgelbe Lösung wird über Nacht stehengelassen und anschließend am Rotationsverdampfer eingeengt.

Man erhält 48,7 g eines schmierigen Feststoffes, der laut ¹H-NMR 11,6 g (77% der Theorie) Trimethyl-(5-methyl-pyridin-2-yl)-ammoniumchlorid enthält.

### Beispiel 5

### (2. Stufe)

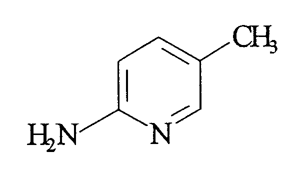

32 g eines nach Umsetzung von 3-Methyl-pyridin-1-oxid mit Trimethylamin und Phosgen anfallenden Rohgemisches, das laut ¹H-NMR 19,3 g (0,035 Mol) Trimethyl-(5-methyl-pyridin-2-yl)-ammoniumchlorid enthält wird mit 35 ml einer 48%igen Bromwasserstofflösung versetzt. Man destilliert das Wasser ab und erhitzt anschließend auf 210°C. Bei dieser Temperatur wird kontinuierlich 48%ige Bromwasserstofflösung eingetropft und Wasser abdestilliert. Nach 8 Stunden ist die Umsetzung laut Dünnschichtchromatographie beendet. Man läßt abkühlen und stellt mit verdünnter Natronlauge auf pH 9 ein. Anschließend wird viermal mit Essigsäureethylester extrahiert. Die vereinigten Extrakte werden über Natriumsulfat getrocknet und nach Filtration am Rotationsverdampfer eingeengt.

Man erhält 9,3 g (83% der Theorie) 2-Amino-5-methylpyridin, das laut gaschromatographischer Analyse 1% 2-Amino-3-methylpyridin enthält.

### Beispiel 6

### (2. Stufe)

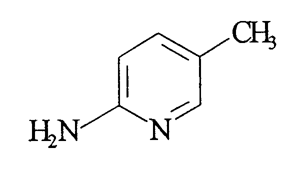

25 g eines nach Umsetzung von 3-Methyl-pyridin-1-oxid mit Trimethylamin und Thionylchlorid anfallenden Rohgemisches, das laut ¹H-NMR 13,06 g (0,07 Mol) Trimethyl-(5-methyl-pyridin-2-yl)-ammoniumchlorid enthält, wird mit 80 ml einer 48%igen Bromwasserstofflösung gemischt und anschließend auf 80°C erhitzt. Nach 1,5 Stunden läßt man abkühlen und gibt unter Eiskühlung 25 ml Pyridin langsam zu. Anschließend werden am absteigenden Kühler bei einer Ölbadtemperatur von 150°C Wasser und Pyridin abdestilliert. Die Ölbadtemperatur wird auf 210°C erhöht. Man läßt 12 Stunden bei dieser Temperatur rühren. Anschließend läßt man abkühlen und stellt mit verdünnter Natronlauge auf pH 9-10 ein. Nach dreimaliger Extraktion mit Essigester werden die vereinigten Extrakte über Natriumsulfat getrocknet, filtriert und am Rotationsverdampfer eingeengt. Im Ölpumpenvakuum werden die Reste an Pyridin entfernt.

Man erhält 6,5 g (86% der Theorie) 2-Amino-5-methylpyridin, das laut gaschromatographischer Analyse ca. 1% 2-Amino-3-methylpyridin enthält.

### Beispiel 7

### (1. + 2. Stufe)

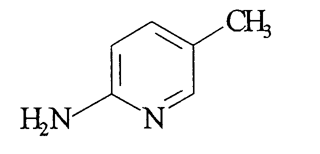

22,2 g (0,377 Mol) Trimethylamin werden bei -10°C kondensiert und bei -5°C zu einer Lösung von 10,03 g (0,092 Mol) 3-Methyl-pyridin-1-oxid in 120 ml Methylenchlorid gegeben. Dann werden bei dieser Temperatur 7,9 ml (0,11 Mol) Thionylchlorid in 15 ml Methylenchlorid innerhalb 30 Minuten eingetropft. Die Temperatur wird während dieses Vorgangs unter 0°C gehalten. Die resultierende gelbe Lösung läßt man auftauen und über Nacht bei Raumtemperatur nachrühren. Im Vakuum wird das Lösungsmittel abgezogen. Anschließend wird mit 35 ml einer 48%igen Bromwasserstofflösung versetzt. Man destilliert das Wasser ab und erhitzt anschließend auf 210°C. Es wird kontinuierlich 48%ige Bromwasserstofflösung eingetropft und Wasser abdestilliert. Nach 8 Stunden ist die Umsetzung laut Dünnschichtchromatographie beendet. Man läßt abkühlen und stellt mit verdünnter Natronlauge auf pH 9 ein. Anschließend wird viermal mit Essigsäureethylester extrahiert. Die vereinigten Extrakte werden über Natriumsulfat getrocknet und nach Filtration am Rotationsverdampfer eingeengt.

Man erhält 8 g (80,5% der Theorie) 2-Amino-5-methylpyridin.

### Beispiel 8

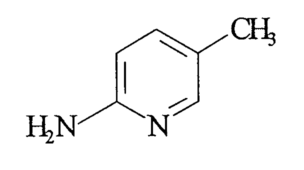

Zu 44 ml 48%iger Bromwasserstoffsäure werden unter Kühlung 4 g (0,029 Mol) 2-Dimethylamino-5-methyl-pyridin gegeben. Ebenfalls unter Kühlung werden 26 ml Pyridin zugesetzt. Man erhitzt das Gemisch 5 Stunden lang auf 150°C (Ölbadtemperatur). Das zunächst vorhandene Wasser wird in dieser Zeit abdestilliert. Anschließend erhöht man die Ölbadtemperatur auf 220°C und läßt weitere 2 Stunden bei dieser Temperatur rühren. Man läßt den Ansatz auf Raumtemperatur abkühlen und gibt das Gemisch sodann in Wasser. Mit Natronlauge wird es auf pH 9 gebracht. Man extrahiert dreimal mit Methylenchlorid, trocknet die vereinigten Extrakte mit Natriumsulfat und engt sie nach Filtration am Rotationsverdampfer ein. Die letzten Reste an Pyridin werden sodann im Ölpumpenvakuum entfernt.

Man erhält 3 g (95% der Theorie) 2-Amino-5-methylpyridin.

### Beispiel 9

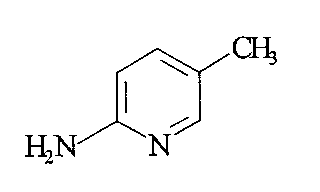

10 g (73,5 mMol) 2-Dimethylamino-5-methyl-pyridin werden unter Eiskühlung in 80 ml zehnprozentiger Salzsäure gelöst. Diese Lösung wird in einem geeigneten Autoklaven gegeben und bis zum Erreichen eines Innendrucks von 30 bar wird Hydrogenchlorid eindosiert. Dann wird die Mischung im verschlossenen Autoklaven unter Rühren auf 180°C erhitzt, wobei der Innendruck auf 70 bar ansteigt. Nach jeweils drei Stunden wird abgekühlt und entspannt. Dann wird erneut Hydrogenchlorid bis zum Erreichen eines Innendrucks von 30 bar eindosiert und wieder auf 180°C erhitzt.

Nach insgesamt 12 Stunden Reaktionszeit wird abgekühlt, entspannt und der Autoklaveninhalt mit Wasser auf etwa das doppelte Volumen verdünnt. Dann wird durch Zugabe von 20%iger Natronlauge der pH-Wert auf 12 eingestellt und mit Essigsäureethylester extrahiert. Die Extraktionslösung wird mit Natriumsulfat getrocknet und filtriert. Vom Filtrat wird dann das Lösungsmittel im Wasserstrahlvakuum sorgfältig abdestilliert.

Man erhält 6,75 g (79% der Theorie) 2-Amino-5-methyl-pyridin (93%ig).

### Zwischenprodukte der Formel (V):

### Beispiel (V-1)

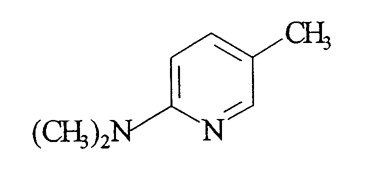

23 g eines nach Umsetzung von 3-Methyl-pyridin-1-oxid mit Trimethylamin und Thionylchlorid anfallenden Rohgemisches, das laut ¹H-NMR 12,69 g (0,068 Mol) Trimethyl-(5-methyl-pyridin-2-yl)-ammoniumchlorid enthält, wird mit 80 ml einer 48%igen Bromwasserstofflösung gemischt und anschließend auf 80°C erhitzt. Nach 1,5 Stunden läßt man abkühlen und stellt mit Natronlauge auf pH 9 ein. Es wird dreimal mit Essigsäureethylester extrahiert.

Nach Trocknen der vereinigten organischen Phasen, Filtration und Entfernen des Lösungsmittels am Rotationsverdampfer erhält man 8,8 g (95% der Theorie) 2-Dimethylamino-5-methylpyridin.
¹H-NMR (300 MHz, CDCl₃):
δ/ppm = 2,17 (3H, Singulett), 3,04 (6H, Singulett), 6,45 (1H, Dublett, J_{AB} = 8,7 Hz), 7,27 (1H, Multiplett, J_{AB} = 8,7 Hz), 7,99 (1H, Quartett, J = 0,9 Hz)

### Beispiel (V-2)

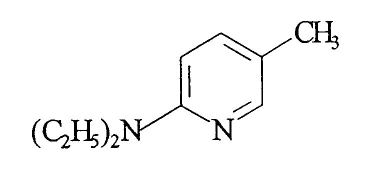

10 g eines nach Umsetzung von 3-Methyl-pyridin-1-oxid mit Triethylamin und Phosgen sowie anschließender Reinigung per Säulenchromatographie anfallenden Rohgemisches, das laut ¹H-NMR-Spektrum 6 g (0,026 Mol) Triethyl-(5-methylpyridin-2-yl)-ammoniumchlorid enthält, werden zu 50 ml verdünnter Natronlauge gegeben und 4 Stunden bei 60°C gerührt. Nach Abkühlen des Gemisches auf Raumtemperatur wird mit Methylenchlorid dreimal extrahiert.

Nach Trocknen der vereinigten Extrakte über Natriumsulfat, Filtration und Entfernen des Lösungsmittels am Rotationsverdampfer erhält man 4,05 g (95% der Theorie) 2-Diethylamino-5-methylpyridin.

### Beispiel (V-3)

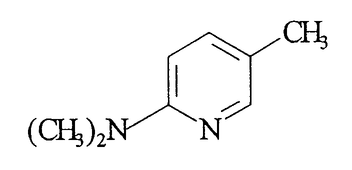

10 g eines bei Umsetzung von 3-Methyl-pyridin-1-oxid mit Trimethylamin und Phosgen entstandenen Rohgemisches, das laut ¹H-NMR 4,5 g (0,024 Mol) Trimethyl-(5-methyl-pyridin-2-yl)-ammoniumchlorid enthält, werden mit ca. 10 ml DMSO versetzt und unter Rühren auf 120°C erhitzt. Man läßt 2 Stunden bei dieser Temperatur rühren. Anschließend wird auf Raumtemperatur abgekühlt und mit Salzsäure auf pH 1 eingestellt. Nach dreimaliger Extraktion mit Methylenchlorid bringt man die wäßrige Phase durch Zugabe von Natronlauge auf pH 12. Es wird erneut dreimal mit Methylenchlorid extrahiert. Die Extrakte der alkalischen Phase werden vereinigt, über Natriumsulfat getrocknet und nach Filtration am Rotationsverdampfer eingeengt.

Man erhält 5,9 g eines Rohgemisches, das laut ¹H-NMR und GC-Analytik 3,05 g 2-Dimethylamino-5-methyl-pyridin (93% der Theorie) enthält.
¹H-NMR: (300 MHz, CDCl₃)
δ/ppm = 2,17 (3H, Singulett), 3,04 (6H, Singulett), 6,45 (1H, Dublett, J_{AB} = 8,7 Hz), 7,27 (1H, Multiplett, J_{AB} = 8,7 Hz), 7,99 (1H, Quartett, J = 0,9 Hz)

## Patentansprüche

1. Verfahren zur Herstellung von 2-Amino-5-methyl-pyridin der Formel (I) 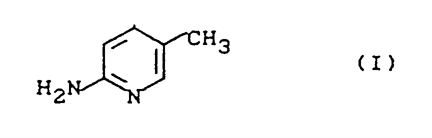 dadurch gekennzeichnet, daß man in einer ersten Stufe 3-Methyl-pyridin-1-oxid der Formel (II) 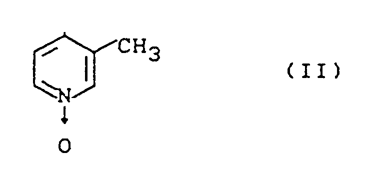 mit einem Trialkylamin der allgemeinen Formel (III)
R₃N (III)
in welcher
R für C₁₋₄-Alkyl steht,
und mit Säurechloriden oder Säureanhydriden als elektrephiler Verbindung, gegebenenfalls in Gegenwart eines Verdünnungsmittels,
zum Ammoniumsalz der allgemeinen Formel (IV) 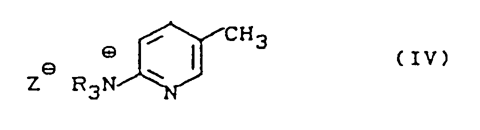 in welcher
R die oben angegebene Bedeutung hat und
Z^{⊖} für ein aus einem Säurechlorid oder Säureanhydrid als elektrophiler Verbindung gebildetes Anion steht, umsetzt,
die Verbindung (IV)
gegebenenfalls als Rohzwischenprodukt isoliert und gegebenenfalls weiter reinigt
und dann in einer zweiten Stufe
a) entweder das Ammoniumsalz der Formel (IV) mit Hydrogenbromid, gegebenenfalls in Gegenwart eines Verdünnungsmittels, bei Temperaturen zwischen 150**°**C und 300**°**C umsetzt,
b) oder alternativ aus der Verbindung (IV) nach üblichen Methoden eine Alkylverbindung RZ abspaltet und das hierbei gebildete 2-Dialkylamino-5-methyl-pyridin der allgemeinen Formel (V) 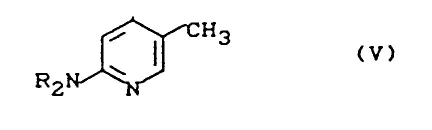 in welcher
R die oben angegebene Bedeutung hat,
mit Hydrogenbromid, gegebenenfalls in Gegenwart eines Verdünnungsmittels, bei Temperaturen zwischen 150**°**C und 300°C umsetzt.

2. Verfahren gemäß Anspruch 1, dadurch gekennzeichnet, daß
R für C₁-C₄-Alkyl
und
Z^{⊖} für ein Chlorid- oder ein C₁-C₄-Alkyl-carboxylation steht.

3. Verfahren gemäß Anspruch 1, dadurch gekennzeichnet, daß
R für Methyl oder Ethyl steht und
Z^{⊖} für ein Chlorid- oder Acetation steht.

4. Verfahren gemäß Anspruch 1 bis 3, dadurch gekennzeichnet, daß R für Methyl steht.

5. Verfahren gemäß Anspruch 1 bis 4, dadurch gekennzeichnet, daß in der ersten Stufe in Gegenwart eines inerten organischen Lösungsmittels als Verdünnungsmittel gearbeitet wird.

6. Verfahren gemäß Anspruch 1 bis 5, dadurch gekennzeichnet, daß, falls Trimethylamin als Trialkylamin (III) eingesetzt wird, die erste Reaktionsstufe im Temperaturbereich zwischen -30°C und +120°C durchgeführt wird.

7. Verfahren gemäß Anspruch 1 bis 5, dadurch gekennzeichnet, daß, falls andere Trialkylamine (III) als Trimethylamin eingesetzt werden, die erste Reaktionsstufe im Temperaturbereich zwischen -30°C und +15°C durchgeführt wird.

8. Verfahren gemäß Anspruch 1 bis 6, dadurch gekennzeichnet, daß man pro Mol 3-Methyl-pyridin-1-oxid (II) zwischen 1 und 10 Mol Trialkylamin (III) und zwischen 1 und 10 Mol Säurechlorid oder Säureanhydrid als elektrophile Verbindung eisetzt.

9. Verfahren gemäß Anspruch 1 bis 7, dadurch gekennzeichnet, daß die zweite Stufe Variante a) in Gegenwart eines Verdünnungsmittels der Reihe Wasser oder organische Lösungsmittel bei Temperaturen zwischen 180 und 250**°**C durchgeführt wird.

10. Verfahren gemäß Anspruch 1 bis 7, dadurch gekennzeichnet, daß man bei der zweiten Stufe, Variante b) die Abspaltung der Alkylverbindung R Z zum Erhalt der Zwischenstufe (V) wie folgt durchführt:
a) Erwärmen mit einer Säure oder
b) Erwärmen mit einer Base oder
c) Erwärmen mit einem Lösungsmittel.

11. 2-Dialkylamino-5-methyl-pyridine der allgemeinen Formel (V) 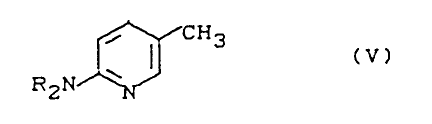 in welcher
R für jeweils identisches C₁₋₄-Alkyl steht.

## Claims

1. Process for the preparation of 2-amino-5-methyl-pyridine of the formula (I) 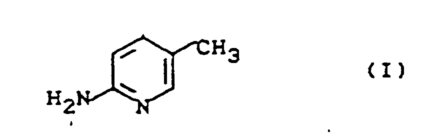 characterised in that in a first step, 3-methyl-pyridine 1-oxide of the formula (II) 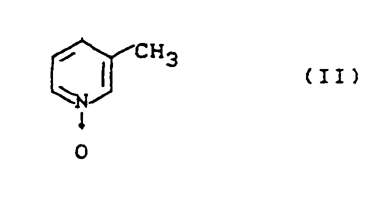 is reacted with a trialkylamine of the general formula (III)
R₃N (III)
in which
R represents C₁₋₄ alkyl,
and with acid chlorides or acid anhydrides as electrophilic compound, in the presence or absence of a diluent,
to give the ammonium salt of the general formula 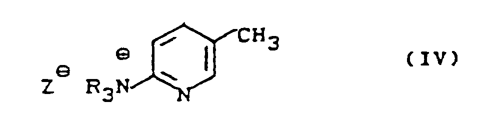 in which
R has the abovementioned meaning and
Z^{⊖} represents an anion formed from an acid chloride or acid anhydride as electrophilic compound,
the compound (IV)
is, possibly, isolated as a crude intermediate and, possibly, further purified
and then in a second step
a) either the ammonium salt of the formula (IV) is reacted with hydrogen bromide, in the presence or absence of a diluent, at temperatures between 150°C and 300°C,
b) or alternatively an alkyl compound RZ is cleaved by conventional methods from the compound (IV) and the 2-dialkylamino-5-methyl-pyridine formed during this of the general formula (V) 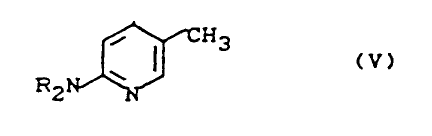 in which
R has the abovementioned meaning,
is reacted with hydrogen bromide, in the presence or absence of a diluent, at temperatures between 150**°**C and 300**°**C.

2. Process according to Claim 1, characterised in that
R represents C₁-C₄-alkyl
and
Z^{⊖} represents a chloride ion or a C₁-C₄-alkyl-carboxylate ion.

3. Process according to Claim 1, characterised in that
R represents methyl or ethyl and
Z^{⊖} represents a chloride ion or acetate ion.

4. Process according to Claim 1 to 3, characterised in that R represents methyl.

5. Process according to Claim 1 to 4, characterised in that the first step is carried out in the presence of an inert organic solvent as diluent.

6. Process according to Claim 1 to 5, characterised in that, if trimethylamine is used as the trialkylamine (III), the first reaction step is carried out in the temperature range between -30°C and +120°C.

7. Process according to Claim 1 to 5, characterised in that if trialkylamines (III) other than trimethylamine are used, the first reaction step is carried out in the temperature range between -30°C and +15°C.

8. Process according to Claim 1 to 6, characterised in that, per mole of 3-methyl-pyridine 1-oxide (II), between 1 and 10 mol of trialkylamine (III) and between 1 and 10 mol of acid chloride or acid anhydride as electrophilic compound are used.

9. Process according to Claim 1 to 7, characterised in that the second step variant a) is carried out in the presence of a diluent selected from the group comprising water or organic solvents at temperatures between 180 and 250°C.

10. Process according to Claim 1 to 7, characterised in that in the second step, variant b), the cleavage of the alkyl compound R Z to obtain the intermediate step (V) is carried out as follows:
a) heating with an acid or
b) heating with a base or
c) heating with a solvent.

11. 2-Dialkylamino-5-methyl-pyridines of the general formula (V) 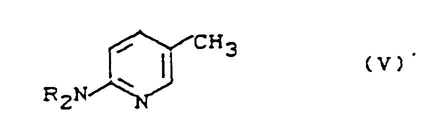 in which
R represents in each case identical C₁₋₄-alkyl.

## Revendications

1. Procédé de production de 2-amino-5-méthyl-pyridine de formule (I) 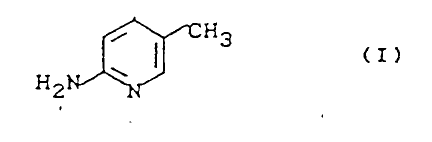 caractérisé en ce que dans une première étape, on fait réagir le 1-oxyde de 3-méthyl-pyridine de formule (II) 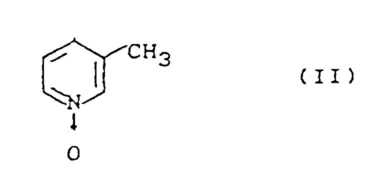 avec une trialkylamine de formule générale (III)
R₃N (III)
dans laquelle
R est un groupe alkyle en C₁ à C₄,
et avec des chlorures d'acides ou des anhydrides d'acides comme composé électrophile, le cas échéant en présence d'un diluant,
pour obtenir le sel d'ammonium de formule générale (IV) 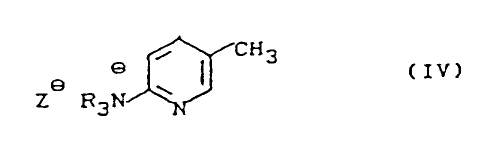 dans laquelle
R a la définition indiquée ci-dessus et
Z^{⊖} représente un anion formé à partir d'un chlorure d'acide ou d'un anhydride d'acide comme composé électrophile,
on isole éventuellement le composé (IV) comme produit intermédiaire brut et on le purifie ensuite, le cas échéant,
puis, dans une seconde étape,
a) on fait réagir le sel d'ammonium de formule (IV) avec le bromure d'hydrogène, éventuellement en présence d'un diluant, à des températures comprises entre 150°C et 300**°**C**,**
b) ou bien en variante, on scinde du composé (IV), par des modes opératoires classiques, un composé alkylique RZ et on fait réagir la 2-dialkylamino-5-méthyl-pyridine ainsi formée, de formule générale (V) 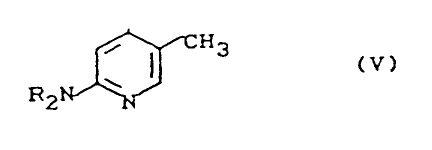 dans laquelle
R a la définition ci-dessus,
avec le bromure d'hydrogène, éventuellement en présence d'un diluant, à des températures comprises entre 150°C et 300°C.

2. Procédé suivant la revendication 1, caractérisé en ce que,
R est un groupe alkyle en C₁ à C₄ et
Z^{⊖} est un ion chlorure ou un ion (alkyle en C₁ à C₄)-carboxylate.

3. Procédé suivant la revendication 1, caractérisé en ce que
R est un groupe méthyle ou éthyle et
Z^{⊖} est un ion chlorure ou acétate.

4. Procédé suivant les revendications 1 à 3, caractérisé en ce que R est le groupe méthyle.

5. Procédé suivant les revendications 1 à 4, caractérisé en ce que, dans la première étape, on opère en présence d'un solvant organique inerte utilisé comme diluant.

6. Procédé suivant les revendications 1 à 5, caractérisé en ce que, lorsqu'on utilise la triméthylamine comme trialkylamine (III), on conduit la première étape de la réaction dans une plage de températures allant de -30°C à +120°C.

7. Procédé suivant les revendications 1 à 5, caractérisé en ce que, lorsqu'on utilise des trialkylamines (III) autres que la triméthylamine, on conduit la première étape de la réaction dans une plage de températures entre -30°C et +15°C.

8. Procédé suivant les revendications 1 à 6, caractérisé en ce qu'on utilise par mole de 1-oxyde de 3-méthyl-pyridine (II) entre 1 et 10 moles de trialkylamine (III) et entre 1 et 10 moles de chlorure d'acide ou d'anhydride d'acide comme composé électrophile.

9. Procédé suivant les revendications 1 à 7, caractérisé en ce qu'on conduit la seconde étape selon la variante a) en présence d'un diluant de la série comprenant l'eau ou des solvants organiques à des températures comprises entre 180 et 250°C.

10. Procédé suivant les revendications 1 à 7, caractérisé en ce que, dans la variante b) de la seconde étape, on conduit la coupure du composé alkylique RZ pour obtenir le stade intermédiaire (V) comme suit :
a) chauffage avec un acide ou
b) chauffage avec une base ou
c) chauffage avec un solvant.

11. 2-dialkylamino-5-méthyl-pyridines de formule générale (V) 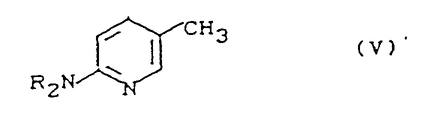 dans laquelle
les groupes R représentent à chaque fois des groupes alkyle en C₁ à C₄ identiques.
